# EUROPÄISCHE PATENTSCHRIFT

(11) **EP 3 373 787 B1**
(45) Veröffentlichungstag und Bekanntmachung des Hinweises auf die Patenterteilung: **23.12.2020**
(21) Anmeldenummer: 16790959.7
(22) Anmeldetag: 31.10.2016
(51) Int. Cl.: A47K 10/48, A61L 2/14, H05H 1/24

(54) **VERFAHREN UND VORRICHTUNG ZUR TROCKNUNG UND PLASMAGESTÜTZTEN DESINFEKTION VON HÄNDEN**
METHOD AND DEVICE FOR THE DRYING AND PLASMA-ASSISTED DISINFECTION OF HANDS
PROCÉDÉ ET DISPOSITIF DE SÉCHAGE ET DE DÉSINFECTION PAR PLASMA DES MAINS

(30) Priorität: 11.11.2015 DE 102015119446
(43) Veröffentlichungstag der Anmeldung: 19.09.2018
(73) Patentinhaber: Hochschule für Angewandte Wissenschaft und Kunst Hildesheim/Holzminden/Göttingen, 31134 Hildesheim (DE)
(72) Erfinder: VIÖL, Wolfgang, 37139 Adelebsen (DE); WIENEKE, Stephan, 37079 Göttingen (DE); TEN BOSCH, Lars, 31174 Schellerten OT Ottbergen (DE); KÖHLER, Robert, 37085 Göttingen (DE); SYRING, Alexander, 37085 Göttingen (DE)
(74) Vertreter: REHBERG HÜPPE + PARTNER
(86) Internationale Anmeldenummer: PCT/EP2016/076251
(87) Internationale Veröffentlichungsnummer: WO 2017/080864

(56) Entgegenhaltungen:
- EP-A1- 2 223 704
- EP-A1- 2 277 424
- US-A1- 2011 277 342
- US-A1- 2013 202 496

## Beschreibung

### TECHNISCHES GEBIET DER ERFINDUNG

Die Erfindung bezieht sich auf ein Verfahren zum Trocknen einer Hand mit einer Handwurzel und Fingerspitzen, wobei die Hand an ihrer Handflächenseite und ihrer Handrückenseite an den Fingerspitzen beginnend mit einem Paar von flächenförmigen Gasstrahlen abgeblasen wird, wobei die Gasstrahlen des Paars in Richtung von der Handwurzel zu den Fingerspritzen unter einem Winkel kleiner als 180° aufeinander zulaufen. Weiterhin bezieht sich die Erfindung auf eine Vorrichtung zum Händetrocknen mit einem eine Händeeinführöffnung aufweisenden Gehäuse und mit zwei an beiden Längsseiten der Händeeinführöffnung angeordneten Schlitzdüsen, die schräg in das Gehäuse hinein ausgerichtet sind, um eine in die Händeeinführöffnung eingeführte Hand mit einem Paar von aus den Schlitzdüsen austretenden flächenförmigen Gasstrahlen an ihrer Handflächenseite und ihrer Handrückenseite abzublasen.

### STAND DER TECHNIK

Ein Verfahren der eingangs beschriebenen Art mit den Merkmalen des Oberbegriffs des unabhängigen Patentanspruchs 1 und eine Vorrichtung der eingangs beschriebenen Art mit den Merkmalen des Oberbegriffs des unabhängigen Patentanspruchs 8 sind aus der WO 2007/015043 A1 bekannt. Die bekannte Vorrichtung zum Händetrocknen weist eine Händeeinführöffnung mit zwei nebeneinander angeordneten, ineinander übergehenden Öffnungsbereichen für jeweils eine Hand auf. Ein Abstand von Längsseiten der Händeeinführöffnung beträgt 70 bis 100 mm, d. h. etwa 85 mm, und verengt sich an den beiden Enden der Händeeinführöffnung und zwischen ihren beiden Öffnungsbereichen auf 50 bis 80 mm, d. h. etwa 65 mm. An den beiden Längsseiten der Händeeinführöffnungen sind Schlitzdüsen angeordnet, die in einem Gehäuse der Vorrichtung an ein Gebläse angeschlossen sind, um flächenförmige Gasstrahlen aus den Schlitzdüsen auszublasen. Die Gasstrahlen bestehen aus Luft, deren Geschwindigkeit wenigsten 80 m/s, vorzugsweise mindestens 100 oder 150 m/s, mehr bevorzugt ungefähr 180 m/s beträgt. Außer der Händeeinführöffnung weist das Gehäuse der bekannten Vorrichtung daran direkt anschließende große seitliche Öffnungen auf, durch die die ausgeblasene Luft der Gasstrahlen in die Umgebung der Vorrichtung gelangt. Damit werden aber auch von den Händen abgeblasenes Wasser und mit dem Wasser von den Händen abgeblasene Keime in der Umgebung der Vorrichtung verteilt. Die von dem Gebläse zum Ausblasen aus den Schlitzdüsen angesaugte Luft wird durch einen Filter angesaugt.

Der Winkel zwischen den aus den Schlitzdüsen austretenden flächenförmigen Gasstrahlen, mit denen die jeweilige Hand an ihrer Handflächenseite und ihrer Handrückenseite abgeblasen wird, ist ein stumpfer Winkel, d. h. die Schlitzdüsen sind jeweils nur wenig schräg in das Gehäuse der Vorrichtung hinein ausgerichtet.

Aus der WO 2014/091191 A1 ist eine Weiterentwicklung der aus der WO 2007/015043 A1 bekannten Vorrichtung zum Händetrocknen bekannt. Hier sind die Schlitzdüsen stärker in das Gehäuse hinein geneigt, wobei der Anstellwinkel der einen Schlitzdüse konstant ist, während der Anstellwinkel der ihr gegenüber liegenden Schlitzdüse über die Breite der Händeeinführöffnung insbesondere in einem Bereich von 50° bis 59° gegenüber einer horizontalen Öffnungsebene variiert.

Eine Vorrichtung zum Händetrocknen mit den Merkmalen des Oberbegriffs des unabhängigen Patentanspruchs 8 ist auch aus der EP 2 277 424 A1 und der US2011/0277342 bekannt.

Aus der EP 2 223 704 A1 ist eine Behandlungsvorrichtung für die Behandlung eines Körperteils eines Patienten mit einem nichtthermischen Plasma bekannt. Insbesondere ist die Vorrichtung zum Sterilisieren einer Hand eines Menschen vorgesehen. Die Vorrichtung weist ein Gehäuse zum vorübergehenden Aufnehmen des Körperteils während der Behandlung und zum Anwenden eines Plasmas auf das Körperteil innerhalb des Gehäuses auf. Das Gehäuse weist eine Einführöffnung zum Einführen des Körperteils in das Gehäuse auf. Das Gehäuse umfasst weiterhin einen Plasmagenerator, einen Hochspannungsgenerator, eine äußere elektrische Isolierung und eine gasdurchlässige Strahlungsabschirmung sowie einen Abstandhalter. Der Plasmagenerator weist zwei im Wesentlichen ebene Anordnungen zur Erzeugung einer dielektrisch behinderten Entladung auf. Die eine Anordnung ist oberhalb, die andere Anordnung unterhalb eines Behandlungsbereichs angeordnet. Jede Anordnung weist zwei Elektroden und eine dielektrischen Barriere zwischen den Elektroden auf. Durch Anlegen einer Wechselhochspannung von dem Hochspannungsgenerator zwischen den beiden Elektroden wird eine dielektrisch behinderte Entladung gezündet, von der ultraviolette Strahlung ausgeht.

Aus der JP 4 796 281 B2 ist eine Vorrichtung zur Desinfektion mithilfe eines heißen Plasmastrahls bekannt. Dazu wird eine dielektrisch behinderte Entladung in einem mit einem Gebläse hervorgerufenen Luftstrom gezündet, die zugleich die gewünschte Wärme erzeugt.

Aus der US 2013/0202496 A1 ist eine Vorrichtung zum Desinfizieren von Händen mit Hilfe von kalten Plasmastrahlen bekannt. Ein Paar von zwei einander gegenüberliegenden Arrays von bei Atmosphärendruck erzeugten kalten Plasmastrahlen wird verwendet, um ein sterilisierendes Volumen zu erzeugen. Eine in dieses Volumen eingeführte Hand wird an ihren Oberflächen desinfiziert. Die einander gegenüberliegenden Arrays aus Plasmastrahlen werden elektrisch 180° außer Phase erzeugt.

Aus der US 8,607,472 B2 ist eine Vorrichtung zum Händetrocknen mit den Merkmalen des unabhängigen Patentanspruchs 8 bekannt, bei der ein lonengenerator aus den Schlitzdüsen austretende flächenförmige Luftstrahlen mit Ionen anreichert, die sterilisierende Wirkung haben. Das von den Händen abgeblasene Wasser wird bei dieser bekannten Vorrichtung aufgefangen, und ein Teil der aus den Schlitzdüsen ausgeblasenen Luft kann von einem an die Schlitzdüsen angeschlossenen Gebläse erneut angesaugt und somit im Kreis geführt werden. Der lonengenerator erzeugt die Ionen mittels einer Korona-Entladung zwischen zwei Elektroden, die stromauf der Schlitzdüse angeordnet sind.

Aus der EP 2 656 762 A2 ist eine Vorrichtung zum Trocknen von Händen mit einem Gehäuse bekannt, in dem ein von außen zugänglicher Hohlraum zur Aufnahme der mittels einer Luftströmung zu trocknenden Hände ausgebildet ist. Weiterhin sind in dem Gehäuse ein Gebläse zur Erzeugung der Luftströmung und ein Mittel zur Verminderung von Keimen in der Luftströmung vorhanden. Bei diesem Mittel zur Verminderung von Keimen kann es sich um eine Einrichtung zur Abgabe einer keimreduzierenden Substanz in die Luftströmung, eine Plasma- oder lonenquelle und/oder eine Strahlungsquelle handeln. Die Plasma- oder lonenquelle kann eine durch Mikrowellen oder Hochfrequenz angeregte Plasma- oder lonenquelle sein. Die Strahlungsquelle kann ein UV-Strahler oder eine dielektrische Barriereentladungslampe sein. Die bekannte Vorrichtung kann weiterhin einen Abluftkanal und einen Zuluftkanal aufweisen, die derart mit dem Hohlraum in Verbindung stehen, dass die Luftströmung durch den Abluft- und den Zuluftkanal sowie den Hohlraum zirkuliert werden kann. Die Mittel zur Verminderung von Keimen sind insbesondere im Bereich des Abluftkanals angeordnet. Sie können aber auch in dem Hohlraum angeordnet sein, beispielsweise dann, wenn es sich um Strahlungsquellen handelt.

Aus der CN 103 876 678 A ist eine Vorrichtung zum Händetrocknen bekannt, die ein Gebläse, einen Heizdraht, eine Elektrodenplatte und eine Wechselspannungsquelle aufweist. Das Gebläse erzeugt mit dem Heizdraht einen warmen Luftstrom auf die Elektrodenplatte. Wenn eine gewaschene Hand auf der Elektrodenplatte ausgestreckt wird, treten Entladungen gegenüber der nassen Oberfläche der Hand auf. Diese töten darauf befindliche Bakterien ab und entfernen Wasser. Zusammen mit dem warmen Luftstrom werden die Hände schnell getrocknet. Die Wechselspannungsquelle stellt eine Wechselspannung zwischen der Elektrodenplatte und einem Gitter bereit, zwischen denen eine Isolatorplatte angeordnet ist. Dabei ist das Gitter dem Heizdraht und dem dahinter liegenden Gebläse zugewandt. Wie bei dieser Anordnung mit der Wechselspannungsquelle eine Entladung zur Erzeugung eines Plasmas gegenüber der nassen Hand erzeugt werden soll, die auf dem Gitter angeordnet werden muss, damit sie zugleich dem warmen Luftstrom ausgesetzt wird, ist nicht offenbart.

Aus der JP 2013-244248 A ist eine Vorrichtung zur Trocknung von Händen mit einem eine Händeeinführöffnung aufweisenden Gehäuse bekannt. Die Händeeinführöffnung führt zwischen Düsenplatten mit einer Vielzahl von Düsen, die schräg in das Gehäuse hinein ausgerichtet sind, um die in das Gehäuse eingeführte Hand abzublasen. Auf den der Hand abgekehrten Rückseiten der Düsenplatten sind Paare von Elektrodenplatten vorgesehen, die mit den Düsen in den Düsenplatten korrespondierende Durchgangslöcher aufweisen. An ihren einander gegenüberliegenden Oberflächen sind die beiden Elektrodenplatten jedes Paars mit einer dielektrischen Beschichtung versehen. Durch Anlegen einer Wechselhochspannung zwischen den Elektroden wird in Luft, die mit einem Gebläse zugeführt wir, ein Plasma gezündet, bevor die Luft durch die Düsenplatten auf die Hand ausgeblasen wird.

### AUFGABE DER ERFINDUNG

Der Erfindung liegt die Aufgabe zugrunde, ein Verfahren zum Trocknen einer Hand und eine Vorrichtung zum Händetrocknen aufzuzeigen, die Keime an der Oberfläche der Hand effizient abtöten und die effizient verhindern, dass Keime von der Hand in die Umgebung verteilt werden.

### LÖSUNG

Die Aufgabe der Erfindung wird durch ein Verfahren zum Trocknen einer Hand mit den Merkmalen des Patentanspruchs 1 und durch eine Vorrichtung zum Händetrocknen mit den Merkmalen des Patentanspruchs 8 gelöst. Bevorzugte Ausführungsformen des Verfahrens und der Vorrichtung sind in abhängigen Patentansprüchen definiert.

### BESCHREIBUNG DER ERFINDUNG

Bei dem erfindungsgemäßen Verfahren zum Trocknen einer Hand mit einer Handwurzel und Fingerspitzen wird die Hand an ihrer Handflächenseite und ihrer Handrückenseite an den Fingerspitzen beginnend mit einem Paar von flächenförmigen Gasstrahlen abgeblasen, wobei die Gasstrahlen des Paars in Richtung von der Handwurzel zu den Fingerspitzen unter einem Winkel kleiner als 180°, vorzugsweise unter einem Winkel zwischen 60° und 150°, aufeinander zulaufen. Weiterhin werden durch Anlegen von Wechselhochspannungspulsen gegenüber Erde an neben beiden flächenförmigen Gasstrahlen angeordnete dielektrisch abgeschirmte Elektroden Direktentladungen über der Hand auf ihrer Handflächenseite und ihrer Handrückenseite hervorgerufen.

Mit den Gasstrahlen, die durch ihr Aufeinanderzulaufen geneigt auf die jeweilige Handflächenseite oder Handrückenseite der Hand auftreffen, wird das meiste anhaftende Wasser von der zu trocknenden Hand abgeblasen. Bei dem erfindungsgemäßen Verfahren ist es jedoch nicht beabsichtigt, die zu trocknende Hand direkt vollständig trocken zu blasen. Vielmehr ist es durchaus erwünscht, dass die Hand zunächst feucht bleibt. Dies erleichtert ganz erheblich das Zünden von Direktentladungen über der Hand durch das Anlegen der Wechselhochspannungspulse gegenüber Erde an die neben den beiden flächenförmigen Gasstrahlen angeordneten dielektrisch abgeschirmten Elektroden. Zudem bewirken diese Direktentladungen eine chemisehe Veränderung oder Aktivierung eines noch auf der Hand befindlichen Feuchtigkeitsfilms, die dem Feuchtigkeitsfilm eine stark keimabtötende Wirkung verleiht. Bevor die Keime, die sich an der Oberfläche der Hand befinden, daher beim Trocknen auch dieses Feuchtigkeitsfilms von der Hand freigesetzt werden, werden sie bei dem erfindungsgemäßen Verfahren abgetötet.

Bei den erfindungsgemäß hervorgerufenen Direktentladungen über der Hand wirkt die Hand als kapazitive Gegenelektrode zu der jeweiligen Elektrode, an die die Wechselhochspannungspulse gegenüber Erde angelegt werden. Das heißt, es tritt keine Gasentladung zwischen den Elektroden auf, an die die Wechselhochspannungspulse angelegt werden, sondern zwischen jeder dieser Elektroden und der Hand. Die Direktentladungen über der Hand sind durch die dielektrische Abschirmung der Elektroden dielektrisch behindert, d. h., die fließenden Ströme sind nur klein. Zudem fließen die bei den Direktentladungen fließenden Ströme mit zunehmender Frequenz der hochfrequenten Wechselhochspannungspulse, die mindestens wenige Kilohertz, vorzugsweise 10 kHz bis mehrere 10 kHz, d. h. bis maximal 200 kHz, und beispielsweise etwa 30 kHz beträgt, aufgrund des Skineffekts ausschließlich an der Oberfläche und nicht im Volumen der Hand. Auch mit dann an der Oberfläche der Hand fließenden Strömen ist ein abtötender Effekt auf die dort befindlichen Keime verbunden. Zugleich wird sichergestellt, dass die Ströme nicht in das Gewebe eindringen und dort Reizungen oder gar Schädigungen hervorrufen. Auch an der Oberfläche der Hand treten bei dem erfindungsgemäßen Verfahren aber weder in unmittelbarer Folge der Direktentladungen über der Hand noch in Folge der dadurch an der Oberfläche der Haut fließenden Ströme signifikante Temperaturerhöhungen der Hand oder ihrer Haut auf. Solchen Temperaturerhöhungen stehen nicht nur die durch die dielektrische Behinderung der Direktentladungen durch die dielektrischen Abschirmungen der Elektroden begrenzten Ströme, sondern auch die auf die Hand gerichteten Gasstrahlen und deren Abkühlungswirkungen entgegen.

Wenn bei den Wechselhochspannungspulsen von ihrer Frequenz gesprochen wird, beziehen sich dabei gemachte Zahlenangaben auf die Pulsfolgefrequenz, mit denen Paare aus einem negativen und einem positiven Spannungspuls aufeinanderfolgen. Die einzelnen Wechselhochspannungspulse, die einzelnen Pulspaare oder Gruppen von Wechselhochspannungspulsen können dabei vergleichsweise große Abstände untereinander aufweisen, so dass ihre Spannungsänderungsraten denjenigen von direkt aufeinanderfolgenden Spannungspulsen mit einer noch viel höheren Frequenz entsprechen. Beispielsweise können die Pulse jedes Pulspaares zueinander einen deutlich kürzeren Zeitabstand aufweisen, als er dem halben Kehrwert der Pulsfolgefrequenz entspricht. Dies erleichtert das Zünden der dielektrisch behinderten Direktentladungen über der Hand mit begrenzten Wechselspannungen von typischerweise wenigen kV bis wenigen 10 kV, insbesondere von 5 kV bis 30 kV.

Die Gasstrahlen, mit denen die Hand bei dem erfindungsgemäßen Verfahren zunächst bis auf einen bewusst belastenden Feuchtigkeitsfilm abgeblasen wird, können insbesondere Luftstrahlen sein. Indem die Gasstrahlen nicht auf direktes Entfernen sämtlicher Feuchtigkeit von der Hand ausgerichtet sind, kann ein Gebläse zum Erzeugen der Luftstrahlen vergleichsweise klein dimensioniert werden, und die mit der Erzeugung und dem Ausblasen der Glasstrahlen verbundene Geräuschentwicklung hält sich ebenfalls in Grenzen. Die über der Hand hervorgerufenen Direktentladungen sind mit keinen relevanten Geräuschen verbunden.

Bei dem erfindungsgemäßen Verfahren können die dielektrisch abgeschirmten Elektroden über die Hand hinweg in einem Abstand gehalten werden, der in Richtung von den Fingerspitzen zu der Handwurzel größer wird. Grundsätzlich kann dazu der Abstand der einander über die Hand hinweg gegenüberliegenden Elektroden verändert werden. Einfacher ist es jedoch, wenn der Abstand der Elektroden in der Richtung von den Fingerspitzen zu der Handwurzel zunimmt, indem diese V-förmig zueinander angestellt werden, oder mehrere Paare von Elektroden mit unterschiedlichen Abständen über die Hand hinweg vorgesehen werden. Insbesondere können die Direktentladungen mit mindestens zwei in Richtung von den Fingerspitzen zu der Handwurzel hintereinander angeordneten Paaren von quer zur Richtung von den Fingerspitzen zu der Handwurzel gestreckt ausgebildeten dielektrisch abgeschirmten Elektroden hervorgerufen werden.

Die Gasstrahlen können insbesondere mit einer Geschwindigkeit ihres Gases gegenüber der Umgebung von 100 bis 350 km/h ausgebildet werden. Das heißt, es handelt sich um durchaus schnelle Gasstrahlen. Die Gasstrahlen sind aber nicht so schnell, wie dies für ein sofortiges Trocknen der Hand erforderlich ist. Hierfür gibt die WO 2007/015043 A1 Geschwindigkeiten von vorzugsweise mindestens 100 oder 150 m/s, mehr bevorzugt ungefähr 180 m/s an, wobei 100 m/s genau 360 km/h und 180 m/s knapp 650 km/h entsprechen.

Vorzugsweise wird die Hand bei dem erfindungsgemäßen Verfahren an ihrer Handflächenseite und ihrer Handrückenseite an den Fingerspitzen beginnend mit mindestens zwei in Richtung von den Fingerspitzen zu der Handwurzel hintereinander angeordneten Paaren von flächenförmigen Gasstrahlen abgeblasen. Dabei laufen die Gasstrahlen jedes Paars in Richtung von der Handwurzel zu den Fingerspitzen unter einem Winkel kleiner als 180° aufeinander zu. So werden insbesondere die Finger der Hand an ihrer Handflächenseite und ihrer Handrückenseite durch zwei Gasstrahlen hintereinander abgeblasen, so dass auch mehr als einen Feuchtigkeitsfilm bildendes Wasser aus den Zwischenräumen der Finger sicher entfernt wird.

Konkret können die Gasstrahlen durch Ausblasen von Gas durch Schlitzdüsen ausgebildet werden, die längs von quer zur Richtung von den Fingerspitzen zu der Handwurzel gestreckt ausgebildeten dielektrisch abgeschirmten Elektroden verlaufen. Dabei kann in dem Gas, aus dem mindestens einer der Gasstrahlen ausgebildet wird, eine zusätzliche dielektrisch behinderte Entladung hervorgerufen werden. Diese zusätzliche dielektrisch behinderte Entladung reichert das Gas, das dann als Gasstrahl auf die Hand geblasen wird, um reaktive Spezies an, die zusätzliche keimabtötende Wirkung haben. Zudem bewirkt die dielektrisch behinderte Entladung in dem Gas eine Entkeimung des Gases selbst.

Bevorzugt wird die dielektrisch behinderte Entladung in dem Gas, aus dem der mindestens eine Gasstrahl ausgebildet wird, zwischen einer längs der jeweiligen Schlitzdüse verlaufenden dielektrisch abgeschirmten Elektrode und einer auf der anderen Seite der Schlitzdüse dazu parallel verlaufenden dielektrisch abgeschirmten Gegenelektrode hervorgerufen. Diese dielektrisch abgeschirmte Gegenelektrode wird dabei vorzugsweise auf Erdpotential gehalten. Sie ist damit anders als die längs der jeweiligen Schlitzdüse verlaufende dielektrisch abgeschirmte Elektrode, an der die Wechselhochspannung gegenüber Erde anliegt, nicht auch zugleich ein Endpunkt einer der Direktentladungen, die über der Hand hervorgerufen werden.

Das von der Hand abgeblasene Wasser wird bei dem erfindungsgemäßen Verfahren vorzugsweise aufgefangen und gesammelt oder abgeführt, wobei enthaltene Keime auf irgendeine bekannte und geeignete Weise abgetötet werden können. In jedem Fall wird verhindert, dass die in dem Wasser enthaltenden Keime durch Zerstäuben oder Verdampfen des Wassers in die Umgebung gelangen.

Durch die unter einem Winkel kleiner als 180° aufeinander zulaufenden Gasstrahlen wird Luft aus der Umgebung mitgerissen. Ein aus dem Abblasen mit den Gasstrahlen resultierender Gasstrom kann damit nur zu einem Teil zur Ausbildung der Gasstrahlen zurückgeführt werden, weil sein Volumen durch die mitgerissene Luft anwächst. Dieser Zuwachs des Gasstroms muss vielmehr kontinuierlich abgeführt werden. Dies geschieht bei dem erfindungsgemäßen Verfahren vorzugsweise durch einen geeigneten Filter, der in diesem Teil des Luftstroms enthaltende Keime zurückhält und/oder abtötet. Vorzugsweise wird in dem Filter zudem Ozon abgebaut, welches durch die Direktentladung über der Hand und eine etwaige zusätzliche dielektrisch behinderte Entladung in dem Gas erzeugt wird. Ein Abbauen des Ozons in dem im Kreislauf geführten des Gasstroms ist hingegen nicht nötig oder gar sinnvoll, weil es hier weiterhin zur Keimabtötung genutzt werden kann.

Eine Vorrichtung zum Händetrocknen mit einem eine Händeeinführöffnung aufweisenden Gehäuse und mit zwei an beiden Längsseiten der Händeeinführöffnung angeordneten Schlitzdüsen, die schräg in das Gehäuse hinein ausgerichtet sind, um eine in die Händeeinführöffnung eingeführte Hand mit einem Paar von aus den Schlitzdüsen austretenden flächenförmigen Gasstrahlen an ihrer Handflächenseite und ihrer Handrückenseite abzublasen, ist erfindungsgemäß dadurch gekennzeichnet, dass neben beiden Schlitzdüsen dielektrisch abgeschirmte Elektroden angeordnet sind und dass eine Wechselhochspannungsquelle an die dielektrisch abgeschirmten Elektroden angeschlossen ist, um Wechselhochspannungspulse gegenüber Erde an die dielektrisch abgeschirmten Elektroden anzulegen, wobei die Wechselhochspannungspulse über der in die Händeöffnung eingeführten Hand Direktentladungen auf ihrer Handflächenseite und ihrer Handrückenseite hervorrufen.

Eine durch die Händeeinführöffnung in das Gehäuse der erfindungsgemäßen Vorrichtung eingeführte Hand wird so sowohl mit den flächenförmigen Gasstrahlen abgeblasen als auch durch die Direktentladungen und durch in deren Folge entstehende reaktive Spezies in einer Weise behandelt, die zum Abtöten von Keimen an der Oberfläche, d. h. der Haut, der Hand führt. Dabei werden durch das Abblasen von Wasser von der Hand, ohne diese sofort vollständig zu trocknen, sowohl besonders gute Voraussetzungen für das Zünden der Direktentladungen als auch für das Entstehen reaktiver Spezies und anderer keimabtötender Bedingungen an der Oberfläche der Hand geschaffen.

Die dielektrisch abgeschirmten Elektroden der erfindungsgemäßen Vorrichtung sind über die Händeeinführöffnung hinweg in einem Abstand gehalten, der in das Gehäuse hinein kleiner wird. Dabei können die Elektroden in das Gehäuse hinein gestreckt ausgebildet und V-förmig zueinander angestellt sein, oder es sind mehrere Elektrodenpaare über die Händeeinführöffnung hinweg in der Richtung in das Gehäuse hinein hintereinander angeordnet. Dabei sind alle diese Elektroden an die Wechselhochspannungsquelle angeschlossen. Grundsätzlich können dabei sämtliche Elektroden an einen einzigen Ausgang der Wechselhochspannungsquelle angeschlossen sein. Für Direktentladungen zwischen der Hand und allen Elektroden ist es jedoch bevorzugt, wenn die Wechselhochspannungsquelle separate Ausgänge oder gar separate Teilquellen für die einzelnen Elektroden aufweist.

Der Abstand der dielektrisch abgeschirmten Elektroden des in Richtung in das Gehäuse hinein ersten Paars kann 5 bis 8 cm betragen, während der Abstand der dielektrisch abgeschirmten Elektroden des in Richtung in das Gehäuse hinein zweiten Paars 2 bis 5 cm betragen kann. Der erste Abstand ist dabei auf die Dicke einer üblichen Hand im Bereich ihrer Handwurzel, der zweite Abstand auf die Dicke einer üblichen Hand im Bereich ihrer Finger abgestimmt.

Vorzugsweise sind bei der erfindungsgemäßen Vorrichtung auch mindestens zwei Paare von schräg aufeinander zu gerichteten und quer zu der Richtung in das Gehäuse hinein verlaufenden Schlitzdüsen hintereinander angeordnet. Konkret kann längs jeder dielektrisch abgeschirmten Elektrode eine Schlitzdüse verlaufen. Dabei kann mindestens einer der dielektrisch abgeschirmten Elektroden über die ihr benachbarte Schlitzdüse hinweg eine dielektrisch abgeschirmte Gegenelektrode auf Erdpotential derart gegenüber liegen, dass die an die dielektrisch abgeschirmte Elektrode angelegten Wechselhochspannungspulse eine dielektrisch behinderte Entladung in der Schlitzdüse hervorrufen. Auf diese Weise enthält bereits der aus der Schlitzdüse austretende flächenförmige Gasstrahl reaktive Spezies, die auf die dielektrisch behinderte Entladung in der Schlitzdüse zurückgehen. Zudem werden Keime in dem Gas des Gasstrahls durch die dielektrisch behinderte Entladung abgetötet.

Vorzugsweise weist die erfindungsgemäße Vorrichtung innerhalb des Gehäuses, in der Richtung in das Gehäuse hinein hinter den Schlitzdüsen eine Auffangrinne für von der Hand abgeblasenes Wasser auf. Über diese Auffangrinne wird das Wasser gesammelt und abgeführt. Auf diese Weise wird verhindert, dass das Wasser zerstäubt wird oder verdampft wird und so zusammen mit den darin enthaltenen Keimen in die Umgebung der Vorrichtung gelangt.

Eine Gasführung in dem Gehäuse der erfindungsgemäßen Vorrichtung kann einen zurück zu den Schlitzdüsen führenden Rückführkanal mit einem darin angeordnetem Gebläse und ein in die Umgebung führenden Abführkanal mit einem darin angeordneten Filter umfassen. Die Gasstrahlen reißen Luft aus der Umgebung mit in das Gehäuse hinein und erhöhen so stetig das bewegte Gasvolumen. Entsprechend kann nicht der gesamte Gas- oder Luftstrom im Kreis geführt werden. Vielmehr muss immer ein Teil zurück in die Umgebung abgeführt werden. Hierfür dient der Abführkanal. Der darin angeordnete Filter kann insbesondere Keime zurückhalten und Ozon abbauen, das in der Vorrichtung durch die Gasentladungen entsteht. Durch den Rückführkanal wird hingegen ein Teil der Luft in der erfindungsgemäßen Vorrichtung zirkuliert und auf diese Weise die von der Vorrichtung in der Umgebung insgesamt hervorgerufene Luftbewegung minimiert.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Dies betrifft auch solche Merkmale, die in separaten Zeichnungen dargestellt sind oder bei deren Beschreibung genannt werden. Diese Merkmale können auch mit unterschiedlichen Patentansprüchen kombiniert werden.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Element die Rede ist, ist dies so zu verstehen, dass genau ein Element, zwei Elemente oder mehr Elemente vorhanden sind.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand eines in den Figuren dargestellten bevorzugten Ausführungsbeispiels weiter erläutert und beschrieben.
- **Fig. 1**: ist ein Querschnitt durch eine erfindungsgemäße Vorrichtung zum Händetrocknen,
- **Fig. 2**: zeigt die Ausbildung einer Schlitzdüse der Vorrichtung gemäß Fig. 1 mit angrenzenden dielektrisch abgeschirmten Elektroden; und
- **Fig. 3**: illustriert die Funktion der erfindungsgemäßen Vorrichtung gemäß Fig. 1.

### FIGURENBESCHREIBUNG

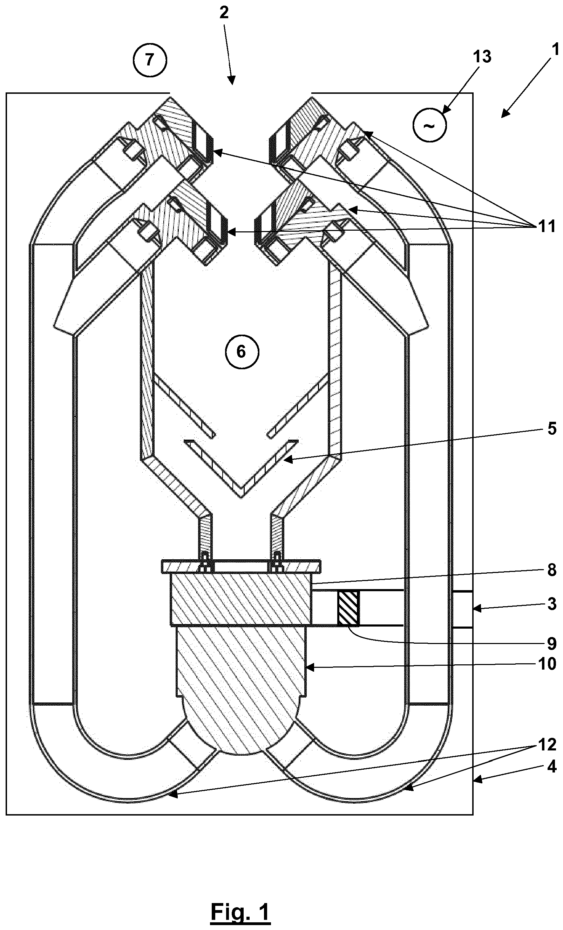

Die in **Fig. 1** dargestellte Vorrichtung 1 umfasst ein bis auf eine Händeeinführöffnung 2 und einen nach außen offenen Abführkanal 3 geschlossenes Gehäuse 4. Durch die Händeeinführöffnung sind zu trocknende Hände zwischen Plasmadüsen 11 einführbar. Die Plasmadüsen 11 liegen sich paarweise über die Händeeinführöffnung 2 hinweg gegenüber, wobei der Abstand der Plasmadüsen 11 des in Richtung in das Gehäuse 4 hinein ersten Paars größer ist, als der Abstand des in dieser Richtung zweiten Paars. Mit den Plasmadüsen 11 werden die in die Händeeinführöffnung 2 eingeführten Hände mit flächenförmigen Gasstrahlen abgeblasen und zugleich mit Plasma behandelt, wie noch detaillierter beschrieben werden wird. Das von den Händen abgeblasene Wasser wird in einer Auffangrinne 5 am Grund eines Auffangraums 6 aufgefangen, in den die Hände mit ihren Fingerspitzen eintreten und der insbesondere das Gas der Gasstrahlen und das mit diesem von den Händen abgeblasene Wasser aufnimmt. Über die Auffangrinne 5 wird das Wasser einem hier nicht dargestellten Sammelbehälter oder Ablauf zugeführt, wobei in dem Sammelbehälter oder dem Ablauf oder in einer dahin führenden Leitung ein Desinfektionsmittel oder Filter zum Zurückhalten und/oder Abtöten von Keimen vorgesehen sein kann. Das Gas der Gasstrahlen und die davon aus der Umgebung 7 der Vorrichtung mitgerissene Luft gelangt bei der Vorrichtung 1 gemäß Fig. 1 insgesamt in einen Filter 8 zum Entfernen von Keimen. Dieser Filter 8 ist aber nur optional. Vorzusehen ist hingegen ein Filter 9 in dem Abführkanal 3, um nicht nur Keime sondern auch Ozon aus der hierüber in die Umgebung 7 abgeführten Abluft herauszufiltern. Der nicht über Abführkanal 3 in die Umgebung 7 ausgeblasene Teil der Luftströmung wird von einem Gebläse 10 über Rückführkanäle 12 zu den Plasmadüsen 11 zurückgeführt. Neben den Rückführkanälen 12 sind die Plasmadüsen 11 über hier nicht dargestellte elektrische Anschlussleitungen an eine Wechselhochspannungsquelle 13 der Vorrichtung 1 angeschlossen. Wenn im Betrieb der Vorrichtung 1 in dem Auffangraum 6 kein Überdruck gegenüber der Umgebung 7 herrscht, ist der Abführkanal 3 mit einem zusätzlichen Gebläse auszustatten, oder er muss stromab des Gebläses 10 abzweigen, um überschüssige Luft aus der Vorrichtung 1 in die Umgebung 7 abführen zu können.

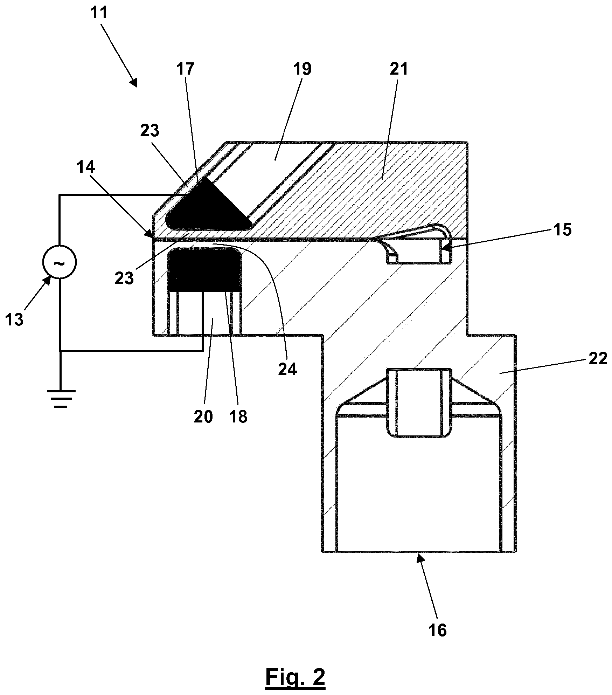

**Fig. 2** zeigt eine der Plasmadüsen 11 gemäß Fig. 1 in vergrößerter separater Darstellung. Die Plasmadüse 11 umfasst eine Schlitzdüse 14, aus der einer der flächenförmigen Gasstrahlen austritt. Die Schlitzdüse 14 ist über eine Vorkammer 15 an einen Gaseinlass 16 der Plasmadüse 11 angeschlossen. Die Spaltweite der Schlitzdüse 14 beträgt hier etwa 0,3 mm. Die Länge der Schlitzdüse senkrecht zur Zeichenebene beträgt hier etwa 260 mm, so dass zwei in dieser Richtung nebeneinander angeordnete Hände mit ihr abgeblasen und plasmabehandelt werden können. Kurz vor der Öffnung der Schlitzdüse 14 in die Umgebung 7 liegen über die Schlitzdüse 14 hinweg zwei Elektroden 17 und 18 einander gegenüber. Dabei sind die Elektroden 17 und 18 in Taschen 19 und 20 von zwei miteinander verbundenen Bauteilen 21 und 22 der Plasmadüse 11 angeordnet. Diese Bauteile 21 und 22 bestehen aus dielektrischem Material. Die Bauteile 21 und 22 bilden somit dielektrische Abschirmungen 23 und 24 der Elektroden 17 und 18.

Mit der Wechselhochspannungsquelle 13 werden hochfrequente Wechselhochspannungspulse zwischen den Elektroden 17 und 18 angelegt, wobei die Elektrode 18 geerdet ist. Diese Wechselhochspannungspulse rufen eine dielektrisch behinderte Entladung zwischen den dielektrischen Abschirmungen 23 und 24 der Elektroden 17 und 18 in der Schlitzdüse 14 hervor. Infolge der dielektrisch behinderten Entladung entsteht ein kaltes Plasma in dem der Schlitzdüse 14 zugeführten Gas, so dass der aus der Schlitzdüse 14 austretende Gasstrahl reaktive Spezies enthält, die Keime abtöten können. Diese reaktiven Spezies töten auch in dem der Schlitzdüse 14 zugeführten Gas enthaltene Keime ab. Überdies ist die dielektrische Abschirmung 23 der Elektrode 17, an der die Wechselhochspannungspulse der Wechselhochspannungsquelle 13 gegenüber Erde anliegt, so ausgebildet, dass zusätzlich Direktentladungen über der in ihre Nähe gebrachten Hand hervorgerufen werden. Die Dicken der dielektrischen Abschirmungen 23 und 24, der Arbeitsabstand der dielektrischen Abschirmungen 23 und 24 über die Schlitzdüse 14 hinweg und die Wechselhochspannungspulse von der Wechselhochspannungsquelle 13 sind so aufeinander abgestimmt, dass die Direktentladungen über der Hand gleichzeitig mit der dielektrisch behinderten Entladung in der Schlitzdüse 14 auftreten. Bei Ausbildung der Bauteile 21 und 22 beispielsweise aus PMMA kann die Dicke der dielektrischen Abschirmung 23 2 mm und die Dicke der dielektrischen Abschirmung 24 1,7 mm betragen. Um die dielektrisch behinderte Entladung in der Schlitzdüse 14 zudem über die gesamte Länge der Schlitzdüse 14 homogen auszubilden, sind die Elektroden 17 und 18 einschließlich ihrer dielektrischen Abschirmungen 23 und 24 mit hoher Parallelität zueinander auszurichten. Zudem ist es für diese Homogenität der dielektrisch behinderten Entladung und der Direktentladungen über der Hand vorteilhaft, wenn zumindest die Elektrode 17 eine raue Oberfläche aufweist. Dies kann beispielsweise durch Ausbilden der Elektroden 17 und 18 aus einem pulverförmigen Ausgangsmaterial realisiert werden. Dieses Ausgangsmaterial, beispielsweise Messingpulver, kann mit Silikon vergossen werden, dass das Ausgangsmaterial vollständig und ohne Zurückbleiben von Lufteinschlüssen benetzt und die Elektroden 17 und 18 durch Auffüllen der Taschen 19 und 20 rückwärtig elektrisch isoliert. Zudem kann für jede Plasmadüse 11 eine separate Teilquelle der Wechselhochspannungsquelle 13 vorgesehen sein, so dass Stromflüsse durch Entladungen im Bereich einer Plasmadüse 11 keine Einflüsse auf die Funktion der anderen Plasmadüsen 11 haben.

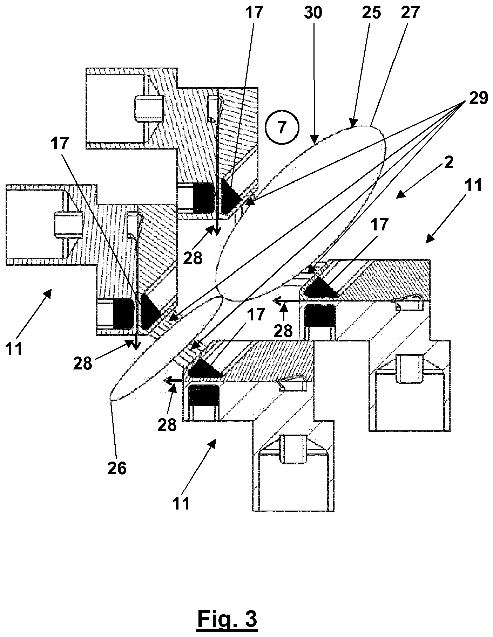

**Fig. 3** zeigt schematisch eine zwischen die vier Plasmadüsen 11 gemäß Fig. 1 eingeführte Hand 25, die sich von Fingerspitzen 26 bis zu einer Handwurzel 27 erstreckt. Die Hand 25 wird an ihrer Handflächenseite und ihrer Handrückenseite mit den aus den Plasmadüsen 11 austretenden flächenförmigen Gasstrahlen 28 abgeblasen, wobei diese Gasstrahlen aufgrund der dielektrisch behinderten Entladungen in den Plasmadüsen 11 bereits reaktive Spezies enthalten, die zur Abtötung von Keimen geeignet sind. Weiterhin bilden sich im Bereich der mit den Wechselhochspannungspulsen gegenüber Erde beaufschlagten Elektroden Direktentladungen 29 über der Hand 25 aus. Diese Direktentladungen 29 werden durch einen noch an der Oberfläche 30, d. h. der Haut, der Hand 25 befindlichen Feuchtigkeitsfilm erleichtert. Zudem modifizieren die Direktentladungen 29 die Zusammensetzung dieses Feuchtigkeitsfilms so, dass eine starke keimabtötende Wirkung resultiert. Insbesondere kann der Feuchtigkeitsfilm durch die Direktentladungen 29 angesäuert werden und eine erhöhte Peroxid- und Nitritkonzentration aufweisen, woraus jeweils keimabtötende Wirkungen resultieren. Die flächenförmigen Gasstrahlen 23 sind paarweise unter einem hier etwa rechten Winkel zueinander ausgerichtet. Die dielektrischen Abschirmungen der mit den Wechselhochspannungspulsen gegenüber Erde beaufschlagten Elektroden, von denen die Direktentladung 29 ausgehen, sind paarweise etwa parallel zueinander und zu der Oberfläche 30 der Hand ausgerichtet. Die Luftgeschwindigkeit in den Gasstrahlen 28 kann knapp 350 km/h betragen. Die Ausgangsspannung der Wechselhochspannungsquelle kann bei etwa 20 kV liegen. Die Wiederholungsfrequenz der Wechselhochspannungspulse kann etwa 30 kHz betragen.

### BEZUGSZEICHENLISTE

- 1: Vorrichtung
- 2: Händeeinführöffnung
- 3: Abführkanal
- 4: Gehäuse
- 5: Auffangrinne
- 6: Auffangraum
- 7: Umgebung
- 8: Filter
- 9: Filter
- 10: Gebläse
- 11: Plasmadüse
- 12: Rückführkanal
- 13: Wechselhochspannungsquelle
- 14: Schlitzdüse
- 15: Vorkammer
- 16: Gasanschluss
- 17: Elektrode
- 18: Elektrode
- 19: Tasche
- 20: Tasche
- 21: Bauteil
- 22: Bauteil
- 23: dielektrische Abschirmung
- 24: dielektrische Abschirmung
- 25: Hand
- 26: Fingerspitze
- 27: Handwurzel
- 28: Gasstrahl
- 29: Direktentladung
- 30: Oberfläche

## Patentansprüche

1. Verfahren zum Trocknen einer Hand (25) mit einer Handwurzel (27) und Fingerspitzen (26),
- wobei die Hand (25) an ihrer Handflächenseite und ihrer Handrückenseite an den Fingerspitzen (26) beginnend mit einem Paar von flächenförmigen Gasstrahlen (28) abgeblasen wird, wobei die Gasstrahlen (28) des Paars in Richtung von der Handwurzel (27) zu den Fingerspitzen (26) unter einem Winkel kleiner als 180° aufeinander zulaufen,
**dadurch gekennzeichnet,**
- **dass** durch Anlegen von hochfrequenten Wechselhochspannungspulsen gegenüber Erde an neben beiden flächenförmigen Gasstrahlen (28) angeordnete dielektrisch abgeschirmte Elektroden (17) Direktentladungen (29) über der Hand (25) auf ihrer Handflächenseite und ihrer Handrückenseite hervorgerufen werden.

2. Verfahren nach Anspruch 1, **dadurch gekennzeichnet, dass** die dielektrisch abgeschirmten Elektroden (17) über die Hand (25) hinweg in einem Abstand gehalten werden, der in Richtung von den Fingerspitzen (26) zu der Handwurzel (27) größer wird, wobei die Direktentladungen (29) optional mit zwei in Richtung von den Fingerspitzen (26) zu der Handwurzel (27) hintereinander angeordneten Paaren von quer zur Richtung von den Fingerspitzen (26) zu der Handwurzel (27) gestreckt ausgebildeten dielektrisch abgeschirmten Elektroden (17) hervorgerufen werden.

3. Verfahren nach einem der vorhergehenden Ansprüche, **dadurch gekennzeichnet, dass** die Hand (25) an ihrer Handflächenseite und ihrer Handrückenseite an den Fingerspitzen (26) beginnend mit zwei in Richtung von den Fingerspitzen (26) zu der Handwurzel (27) hintereinander angeordneten Paaren von flächenförmigen Gasstrahlen (28) abgeblasen wird, wobei die Gasstrahlen (28) jedes Paars in Richtung von der Handwurzel (27) zu den Fingerspitzen (26) unter einem Winkel kleiner als 180° aufeinander zulaufen.

4. Verfahren nach einem der vorhergehenden Ansprüche, **dadurch gekennzeichnet, dass** die Gasstrahlen (28) durch Ausblasen von Gas durch Schlitzdüsen (14) ausgebildet werden, die längs von quer zur Richtung von den Fingerspitzen (26) zu der Handwurzel (27) gestreckt ausgebildeten dielektrisch abgeschirmten Elektroden (17) verlaufen, und dass in dem Gas, aus dem mindestens einer der Gasstrahlen (28) ausgebildet wird, eine dielektrisch behinderte Entladung hervorgerufen wird.

5. Verfahren nach Anspruch 4, **dadurch gekennzeichnet, dass** die dielektrisch behinderte Entladung zwischen der längs der jeweiligen Schlitzdüse (14) verlaufenden dielektrisch abgeschirmten Elektrode (17) und einer auf der anderen Seite der Schlitzdüse (14) dazu parallel verlaufenden dielektrisch abgeschirmten Gegenelektrode (18) hervorgerufen wird, wobei optional die dielektrisch abgeschirmte Gegenelektrode (18) auf Erdpotential gehalten wird.

6. Verfahren nach einem der vorhergehenden Ansprüche, **dadurch gekennzeichnet, dass** die Gasstrahlen (28) mit einer Geschwindigkeit ihres Gases gegenüber der Umgebung (7) von 100 bis 350 km/h ausgebildet werden und dass von der Hand (25) abgeblasenes Wasser aufgefangen wird.

7. Verfahren nach einem der vorhergehenden Ansprüche, **dadurch gekennzeichnet, dass** ein aus dem Ablasen mit den Gasstrahlen (28) resultierender Gasstrom zu einem Teil zur Ausbildung der Gasstrahlen (28) zurückgeführt und zu einem anderen Teil durch einen Filter (9) in die Umgebung (7) abgeführt wird.

8. Vorrichtung (1) zum Händetrocknen,
- mit einem eine Händeeinführöffnung (2) aufweisenden Gehäuse (4) und
- mit zwei an beiden Längsseiten der Händeeinführöffnung (2) angeordneten Schlitzdüsen (14), die schräg in das Gehäuse (4) hinein ausgerichtet sind, um eine in die Händeeinführöffnung (2) eingeführte Hand mit einem Paar von aus den Schlitzdüsen (14) austretenden flächenförmigen Gasstrahlen (28) an ihrer Handflächenseite und ihrer Handrückenseite abzublasen,
**dadurch gekennzeichnet,**
- **dass** neben beiden Schlitzdüsen (14) dielektrisch abgeschirmte Elektroden (17) angeordnet sind und
- **dass** eine Wechselhochspannungsquelle (13) an die dielektrisch abgeschirmten Elektroden (17) angeschlossen ist, um hochfrequente Wechselhochspannungspulse gegenüber Erde anzulegen, die über der in die Händeeinführöffnung (2) eingeführten Hand (25) Direktentladungen (29) auf ihrer Handflächenseite und ihrer Handrückenseite hervorrufen.

9. Vorrichtung (1) nach Anspruch 8, **dadurch gekennzeichnet, dass** die dielektrisch abgeschirmten Elektroden (17) über die Händeeinführöffnung (2) hinweg in einem Abstand gehalten sind, der in das Gehäuse (4) hinein kleiner wird, wobei die dielektrisch abgeschirmten Elektroden (17) in Richtung in das Gehäuse hinein gestreckt ausgebildet und V-förmig zueinander angestellt sind.

10. Vorrichtung (1) nach Anspruch 8, **dadurch gekennzeichnet, dass** in Richtung in das Gehäuse (4) hinein zwei Paare von quer zur Richtung in das Gehäuse (4) hinein gestreckt ausgebildeten und einander über die Händeeinführöffnung (2) hinweg gegenüber liegenden dielektrisch abgeschirmten Elektroden (17) hintereinander angeordnet sind, die an die Wechselhochspannungsquelle (13) angeschlossen sind, wobei optional der Abstand der dielektrisch abgeschirmten Elektroden (17) des in Richtung in das Gehäuse (4) hinein ersten Paars 5 cm bis 8 cm und der Abstand der dielektrisch abgeschirmten Elektroden (17) des in Richtung in das Gehäuse hinein zweiten Paars 2 cm bis 5 cm beträgt.

11. Vorrichtung (1) nach einem der Ansprüche 8 bis 10, **dadurch gekennzeichnet, dass** in Richtung in das Gehäuse (4) hinein zwei Paare von schräg aufeinander zu gerichteten und quer zur Richtung in das Gehäuse (4) hinein verlaufenden Schlitzdüsen hintereinander angeordnet sind.

12. Vorrichtung (1) nach einem der Ansprüche 8 bis 11, **dadurch gekennzeichnet, dass** mindestens einer der dielektrisch abgeschirmten Elektroden (17) über eine der Schlitzdüsen (14) hinweg, die der mindestens einen der dielektrisch abgeschirmten Elektroden (17) benachbart ist, eine dielektrisch abgeschirmte Gegenelektrode (18) auf Erdpotential derart gegenüber liegt, dass die an die dielektrisch abgeschirmte Elektrode (17) angelegten Wechselhochspannungspulse eine dielektrisch behinderte Entladung in der Schlitzdüse (14) hervorrufen.

13. Vorrichtung (1) nach einem der Ansprüche 8 bis 12, **dadurch gekennzeichnet, dass** in Richtung in das Gehäuse (4) hinein hinter den Schlitzdüsen (14) eine Auffangrinne (5) für von der Hand abgeblasenes Wasser in dem Gehäuse (4) angeordnet ist.

14. Vorrichtung (1) nach einem der Ansprüche 8 bis 13, **dadurch gekennzeichnet, dass** eine Gasführung in dem Gehäuse (4) einen zurück zu den Schlitzdüsen (14) führenden Rückführkanal (12) mit einem darin angeordneten Gebläse (10) und einen in die Umgebung führenden Abführkanal (3) mit einem darin angeordneten Filter (9) umfasst.

15. Vorrichtung (1) nach einem der Ansprüche 8 bis 14, **dadurch gekennzeichnet, dass** die Wechselhochspannungsquelle (13) für jede der dielektrisch abgeschirmten Elektroden (17) eine Teilquelle zum Anlegen der Wechselhochspannungspulse an die jeweilige dielektrisch abgeschirmte Elektrode (17) aufweist.

## Claims

1. Method of drying a hand (25) having a carpus (27) and fingertips (26),
- wherein the hand (25) is blown-off at its palm side and its back side beginning at the fingertips (26) with a pair of planar gas jets (28), wherein the gas jets (28) of the pair run towards each other in direction from the carpus (27) to the fingertips (26) at an angle of less than 180°,
**characterized in**
- **that** direct discharges (29) on the hand (25) are generated on its palm side and its back side by applying high frequent alternating high voltage pulses with regard to ground to dielectrically shielded electrodes (17) arranged next to both planar gas jets (28).

2. Method of claim 1, **characterized in that** the dielectrically shielded electrodes (17) are held at a distance across the hand (25), the distance increasing in direction from the fingertips (26) to the carpus (27), wherein the direct discharges (29) are optionally generated with two pairs of the dielectrically shielded electrodes (17) extending transverse to the direction from the fingertips (26) to the carpus (27), the two pairs of the dielectrically shielded electrodes (17) being arranged one behind the other in the direction from the fingertips (26) to the carpus (27).

3. Method of any of the preceding claims, **characterized in that** the hand (25) is blown-off at its palm side and its back side beginning at the fingertips (26) with two pairs of planar gas jets (28) which are arranged one behind the other in direction from the fingertips (26) to the carpus (27), wherein the gas jets (28) of each pair run towards each other in direction from the carpus (27) to the fingertips (26) at an angle of less than 180°.

4. Method of any of the preceding claims, **characterized in that** the gas jets (28) are formed by blowing gas through slot nozzles (14) which run along dielectrically shielded electrodes (17) elongated transverse to the direction from the fingertips (26) to the carpus (27), and that a dielectric barrier discharge is generated in a gas of which at least one of the gas jets (28) is formed.

5. Method of claim 4, **characterized in that** the dielectric barrier discharge is generated between the dielectrically shielded electrode (17) running along the respective slot nozzle (14) and a dielectrically shielded counter electrode (18) running in parallel thereto on the other side of the slot nozzle (14), wherein the dielectrically shielded counter electrode (18) is optionally held on ground potential.

6. Method of any of the preceding claims, **characterized in that** the gas jets (28) are provided at a velocity of their gas of 100 to 350 km/h with regard to their surroundings (7), and that water blown of the hand (25) is collected.

7. Method of any of the preceding claims, **characterized in that** a part of a gas stream resulting from blowing off with the gas jets (28) is in one part recirculated for forming the gas jets (28) and in another part discharged through a filter (9) into the surroundings (7).

8. Device (1) for hand drying,
- comprising a housing (4) having a hand insertion opening (2), and
- comprising two slot nozzles (14) arranged on both longitudinal sides of the hand insertion opening (2), which are oriented into the housing (4) at a slant angle to blow off a palmar side and a back side of a hand inserted into the hand insertion opening (2) with a pair of planar gas jets (28) emerging out of the slot nozzles (14),
**characterized in**
- **that** dielectrically shielded electrodes (17) arranged next to the slot nozzles (14), and
- **that** an alternating high voltage source (13) is connected to the dielectrically shielded electrodes (17) to apply high frequency alternating high voltage pulses with regard to ground which generate direct discharges (29) on the hand (25) inserted into the hand insertion opening (2) on its palmar side and its back side.

9. Device (1) of claim 8, **characterized in that** the dielectrically shielded electrodes (17) are held at a distance across the hand insertion opening (2), the distance decreasing in a direction into the housing (4), wherein the dielectrically shielded electrodes (17) are elongated in direction into the housing and inclined with regard to another in a V-shape.

10. Device (1) of claim 8, **characterized in that**, in direction into the housing (4), two pairs of dielectrically shielded electrodes (17) elongated transverse to the direction into the housing (4) and facing each other across the hand insertion opening (2), which are connected to the alternating high voltage source (13), are arranged one after the other, and wherein, optionally, the distance between the dielectrically shielded electrodes (17) of the first pair in direction into the housing (4) is 5 cm to 8 cm, and the distance between the dielectrically shielded electrodes (17) of the second pair in direction into the housing (4) is 2 cm to 5 cm.

11. Device (1) of any of the claims 8 to 10, **characterized in that**, in direction into the housing (4), two pairs of slot nozzles which are oriented at a slant angle towards each other and are running traverse to the direction into the housing (4) are arranged one behind the other.

12. Device (1) of any of the claims 8 to 11, **characterized in that** at least one of the dielectrically shielded electrodes (17) is facing a dielectrically shielded counter electrode (18) held on ground potential across one of the slot nozzles (14) neighbouring the at least one of the dielectrically shielded electrodes (17) such that the alternating high voltage pulses applied to the dielectrically shielded electrode (17) cause a dielectric barrier discharge in the slot nozzle (14).

13. Device (1) of any of the claims 8 to 12, **characterized in that**, in direction into the housing (4), behind the slot nozzles (14) a collection gutter (5) for water blown of the hand is arranged in the housing (4).

14. Device (1) of any of the claims 8 to 13, **characterized in that** a gas guidance within the housing (4) has a recirculation channel (12) guiding back to the slot nozzles (14), a blower (10) arranged in the recirculation channel, and an discharge channel (3) guiding into the surroundings and having a filter (9) arranged therein.

15. Device (1) of any of the claims 8 to 14, **characterized in that** the alternating high voltage source (13) has a partial source for each of the dielectrically shielded electrodes (17) for applying the alternating high voltage pulses to the respective dielectrically shielded electrode (17).

## Revendications

1. Procédé de séchage d'une main (25) comportant un carpe (27) et des bouts de doigts (26),
- la main (25) étant balayée par une paire de jets gazeux (28) plats au niveau de son côté paume de main et de son côté dos de main, en commençant par les bouts de doigts (26), les jets gazeux (28) de la paire convergeant l'un vers l'autre dans la direction allant du carpe (27) vers les bouts de doigts (26) en formant un angle inférieur à 180°,
**caractérisé en ce que,**
- l'application d'impulsions haute tension alternatives haute fréquence par rapport à la terre sur des électrodes (17) à protection diélectrique disposées près des deux jets gazeux (28) plats provoque des décharges directes (29) sur la main (25) au niveau de son côté paume et de son côté dos.

2. Procédé selon la revendication 1, **caractérisé en ce que** les électrodes (17) à protection diélectrique sont retenues par-dessus la main (25) à une distance qui grandit dans la direction allant des bouts de doigts (26) vers le carpe (27), les décharges directes (29) étant provoquées optionnellement par deux paires, disposées l'une derrière l'autre dans la direction allant des bouts de doigts (26) vers le carpe (27), d'électrodes (17) à protection diélectrique constituées de façon étendue transversalement à la direction allant des bouts de doigts (26) vers le carpe (27).

3. Procédé selon l'une des revendications précédentes, **caractérisé en ce que** la main (25) est balayée par deux paires, disposées l'une derrière l'autre dans la direction allant des bouts de doigts (26) vers le carpe (27), de jets gazeux (28) plats au niveau de son côté paume de main et de son côté dos de main, en commençant par les bouts de doigts (26), les jets gazeux (28) de chaque paire convergeant l'un vers l'autre dans la direction allant du carpe (27) vers les bouts de doigts (26) en formant un angle inférieur à 180°.

4. Procédé selon l'une des revendications précédentes, **caractérisé en ce que** les jets gazeux (28) sont constitués par le soufflage de gaz à travers des buses en forme de fente (14) qui sont placées le long d'électrodes (17) à protection diélectrique constituées de façon étendue transversalement à la direction allant des bouts de doigts (26) vers le carpe (27), et **en ce qu'**une décharge à barrière diélectrique est provoquée dans le gaz dont au moins un des jets gazeux (28) est constitué.

5. Procédé selon la revendication 4, **caractérisé en ce que** la décharge à barrière diélectrique est provoquée entre l'électrode (17) à protection diélectrique placée le long de la buse en forme de fente (14) respective et une contre-électrode (18) à protection diélectrique placée sur l'autre côté de la buse en forme de fente (14) parallèlement à celle-ci, la contre-électrode (18) à protection diélectrique étant optionnellement maintenue au potentiel de la terre.

6. Procédé selon l'une des revendications précédentes, **caractérisé en ce que** les jets gazeux (28) sont constitués avec une vitesse de leur gaz par rapport à l'environnement (7) de 100 à 350 km/h, et **en ce que** l'eau enlevée de la main (25) par soufflage est collectée.

7. Procédé selon l'une des revendications précédentes, **caractérisé en ce qu'**un flux de gaz résultant du soufflage avec les jets gazeux (28) est pour une part recyclé pour la constitution des jets gazeux (28) et pour une autre part évacué dans l'environnement (7) à travers un filtre (9).

8. Dispositif (1) de séchage des mains,
- avec un boîtier (4) comportant une ouverture d'introduction de main (2) et
- avec deux buses en forme de fente (14) qui sont disposées sur les deux côtés longitudinaux de l'ouverture d'introduction de main (2) et qui sont orientées obliquement vers l'intérieur du boîtier (4) pour balayer, sur son côté paume de main et son côté dos de main, une main introduite dans l'ouverture d'introduction de main (2) avec une paire de jets gazeux (28) plats sortant des buses en forme de fente (14),
**caractérisé en ce que**
- des électrodes (17) à protection diélectrique sont disposées près des deux buses en forme de fente (14), et
- **en ce qu'**une source de haute tension alternative (13) est connectée aux électrodes (17) à protection diélectrique pour appliquer des impulsions haute tension alternatives haute fréquence par rapport à la terre qui provoquent, au-dessus de la main (25) introduite dans l'ouverture d'introduction de main (2), des décharges directes (29) sur son côté paume de main et son côté dos de main.

9. Dispositif (1) selon la revendication 8, **caractérisé en ce que** les électrodes (17) à protection diélectrique sont retenues par-dessus l'ouverture d'introduction de main (2) à une distance qui diminue vers l'intérieur du boîtier (4), les électrodes (17) à protection diélectrique étant constituées de façon étendue en direction de l'intérieur du boîtier et étant placées l'une par rapport à l'autre en formant un V.

10. Dispositif (1) selon la revendication 8, **caractérisé en ce que,** en direction de l'intérieur du boîtier (4), deux paires d'électrodes (17) à protection diélectrique, constituées de façon étendue transversalement à la direction vers l'intérieur du boîtier (4) et opposées l'une à l'autre par-dessus l'ouverture d'introduction de main (2), sont disposées l'une derrière l'autre, et sont connectées à la source de haute tension alternative (13), la distance des électrodes (17) à protection diélectrique de la première paire en direction de l'intérieur du boîtier (4) étant optionnellement de 5 cm à 8 cm, et la distance des électrodes (17) à protection diélectrique de la deuxième paire en direction de l'intérieur du boîtier étant de 2 cm à 5 cm.

11. Dispositif (1) selon l'une des revendications 8 à 10, **caractérisé en ce que,** en direction de l'intérieur du boîtier (4), deux paires de buses en forme de fente dirigées obliquement l'une par rapport à l'autre et placées transversalement à la direction vers l'intérieur du boîtier (4) sont disposées l'une derrière l'autre.

12. Dispositif (1) selon l'une des revendications 8 à 11, **caractérisé en ce que,** par rapport à au moins une des électrodes (17) à protection diélectrique, par-dessus une des buses en forme de fente (14) qui est proche d'au moins l'une des électrodes (17) à protection diélectrique, une contre-électrode (18) à protection diélectrique est au potentiel de la terre de telle sorte que les impulsions haute tension alternatives appliquées à l'électrode (17) à protection diélectrique provoquent une décharge à barrière diélectrique dans la buse en forme de fente (14).

13. Dispositif (1) selon l'une des revendications 8 à 12, **caractérisé en ce que,** en direction de l'intérieur du boîtier (4) derrière les buses en forme de fente (14), une rigole de collecte (5) pour l'eau enlevée de la main par soufflage est disposée dans le boîtier (4).

14. Dispositif (1) selon l'une des revendications 8 à 13, **caractérisé en ce qu'**une conduite de gaz dans le boîtier (4) comprend un canal de retour (12) ramenant aux buses en forme de fente (14), avec un ventilateur (10) disposé à l'intérieur, et un canal d'évacuation (3) conduisant dans l'environnement, avec un filtre (9) disposé à l'intérieur.

15. Dispositif (1) selon l'une des revendications 8 à 14, **caractérisé en ce que** la source de haute tension alternative (13) pour chacune des électrodes (17) à protection diélectrique comporte une source partielle pour l'application des impulsions haute tension alternatives à l'électrode (17) à protection diélectrique respective.
